Europäisches Patentamt

European Patent Office

Office européen des brevets

(19)

(11) Publication number: **0 155 832**
**A2**

(12) # EUROPEAN PATENT APPLICATION

(21) Application number: **85301829.9**

(22) Date of filing: **15.03.85**

(51) Int. Cl.⁴: **C 12 P 21/00**
**C 07 K 3/00, C 12 N 15/00**

(30) Priority: **16.03.84 US 590121**
**11.09.84 US 649448**

(43) Date of publication of application:
**25.09.85 Bulletin 85/39**

(84) Designated Contracting States: ·
**AT BE CH DE FR GB IT LI LU NL SE**

(71) Applicant: **GENENTECH, INC.**
**460 Point San Bruno Boulevard**
**South San Francisco California 94080(US)**

(72) Inventor: **Wetzel, Ronald Burnell**
**455, Urbano Drive**
**San Francisco California 94127(US)**

(74) Representative: **Armitage, Ian Michael et al,**
**MEWBURN ELLIS & CO. 2/3 Cursitor Street**
**London EC4A 1BQ(GB)**

(54) Stable proteins, methods for their construction, plasmids and other DNA encoding them, cell cultures harboring such plasmids.

(57) Proteins are mutated in order to introduce disulfide linkages at sites not normally found in the wild type protein. Additional disulfide bonds formed in this fashion stabilize the proteins from inactivation.

EP 0 155 832 A2

Croydon Printing Company Ltd

1

STABLE PROTEINS, METHODS FOR THEIR CONSTRUCTION,

PLASMIDS AND OTHER DNA ENCODING THEM, CELL

CULTURES HARBORING SUCH PLASMIDS

The present invention relates to the field of protein engineering.

Proteins are one of a number of biologically functional chemical compounds. Proteins are unusual among biochemical compounds in their ability to catalyze reactions. Catalytic proteins are called enzymes.

All proteins are comprised of combinations of twenty monomer building blocks called amino acids. The amino acids are linearly linked together by a special type of covalent bond termed the peptide bond. The order or sequence of the amino acids so linked is the primary structure of a protein. The primary structure is important for at least two reasons. First, it determines the three-dimensional conformation of a protein.

Second, the primary structure is a direct linear translation of the DNA nucleotides. Thus, essential information regarding the sequence of DNA nucleotides can be ascertained from the primary structure of a protein and vice versa.

The three-dimensional interaction of a protein comprises its secondary and tertiary structure. Secondary structure is the relationship or interaction between neighboring amino acids of the primary chain. Tertiary structure is the relationship or interaction between amino acids at some distance from each other in the primary chain. Owing to secondary and tertiary structure many complex interactions may occur between amino acids separated from each other within the primary sequence. The result of these interactions is protein folding. This can be envisioned as a string of bar magnets with each individual bar magnet representing an amino acid and the entire string of magnets representing a protein. If the string of magnets is tossed into the air it will assume one of many possible conformations limited by the physical properties of the magnets i.e. like magnetic poles repel so a three-dimensional conformation cannot exist with like poles next to each other.

Disulfide bonds aid in maintaining the tertiary structure of proteins. Whatever the conformation, a change in the environment of a protein may alter the forces exerted on the protein, with a concommitant change in the threedimensional conformation of the protein. For instance, the application of heat to a protein results in a conformational change. If a high temperature is applied the protein may not revert to its original conformation even on cooling.

Enzymes are proteins that catalyze biological reactions. Enzymes are highly substrate specific. Biological reactions are catalyzed in the active site of an enzyme. The enzyme active site is specific for designated chemical reactants. Frequently, the enzyme is so specific that only one chemical can react. An important factor in this catalytic rate enhancement and specificity is the three-dimensional folding of enzymes. A disruption or change in the three-dimensional conformation of enzymes usually causes a loss or lessening of catalytic activity. Such a change of catalytic activity via a folding change is termed denaturation. Depending on the extent and nature of the three-dimensional change or disruption the loss or lessening of catalytic activity can be reversible or irreversible.

Some proteins which have the amino acid cysteine in their primary structure form an additional covalent bond between cysteines. This covalent crosslinking bond is called a disulfide bond. A disulfide bond can only form between two cysteines since cysteine is the only amino acid with a free sulfhydryl group (-SH). As noted earlier, disulfide bonds are the only covalent bonds besides peptide bonds which aid in maintaining tertiary structure. Other non-covalent interactions are involved in protein stabilization as well.

No discussions are extant in the literature which directly address the prospects for inserting disulfide bonds into proteins without destroying their active structure. This is probably because it is only very recently (1, 2) that it has become possible to make homogeneous protein analogs containing the cysteine replacements required as a first step in such a process.

Nonetheless, there are literature discussions (3, 4) which imply such difficulty. The study of the detailed structures of disulfide bonds, in proteins whose x-ray cystal structures have been determined, indicates that these bonds exist in only a few types of configurations, as defined by the values of five dihedral angles between various atoms in the disulfide connected cysteine side chains. When one attempts to construct by computer graphics terminal, a disulfide between two cysteines inserted at favorable sites in a protein structure, one finds that the dihedral angles obtained generally differ considerably from those actually observed in disulfide containing proteins.

Much remains to be learned about the mechanism by which disulfide bonds influence conformational stability or the pathways of protein folding or unfolding. The nature of the stability achieved in known examples is varied and may include stability against heat, denaturants, heating and cooling, as well as hydrolysis by enzymes. On the other hand, Villafranca, et al (5) have reported that the addition of disulfide bonds may so reduce "dynamic flexibility" that protein activity should not be expected.

Prior to the present invention, only scientific speculation attended the possible advantages of introducing disulfide bonds because the technical problems of introducing a disulfide bond were viewed as insurmountable. Furthermore, it was unclear what effect the addition of a disulfide bond would have to a protein which is otherwise stable without such disulfide bonds. Three of the more commonly acknowledged roadblocks were (1) the fundamental problem of why, over the long course of evolution, there were not more disulfide bonds found in proteins given their apparent advantages, (2) the problem of whether the protein's folding pattern could accommodate a disulfide bond given the restrictive dihedral angles in protein

5

disulfides and, if it could, whether the protein would still function and; (3) the technical problem of constructing a synthetic polypeptide which would permit the introduction of the amino acid cysteine.

The present invention successfully overcomes the problem of introduction of disulfide bonds into proteins which may or may not contain disulfide bonds in their native or wild type. It has been found that the addition of disulfide bonds into appropriate positions leads to protein stabilization against heat and should also stabilize proteins from inactivating forces such as pH extremes, presence of denaturing chemicals, oxidation, hydrolysis, temperature variations or degradative enzymes.

The present invention provides a method for the introduction of disulfide bonds into proteins, regardless of whether the protein possesses disulfide bonds in its natural or wild type state, while permitting the protein to retain activity. The present invention also provides proteins including additional disulfide bonds.

The newly created disulfide bonded proteins made available by this invention are useful in a variety of settings including the pharmacological applications of increased hormone stability and in commercial applications of increased enzyme stability in harsh environments. Overall biological effect is not affected in kind. In a preferred embodiment, the invention represents the first occasion in which disulfide bonds have been

6

specifically introduced into proteins while retaining protein activity.

The figures illustrate the preferred embodiment, described in section F below, by way of example only.

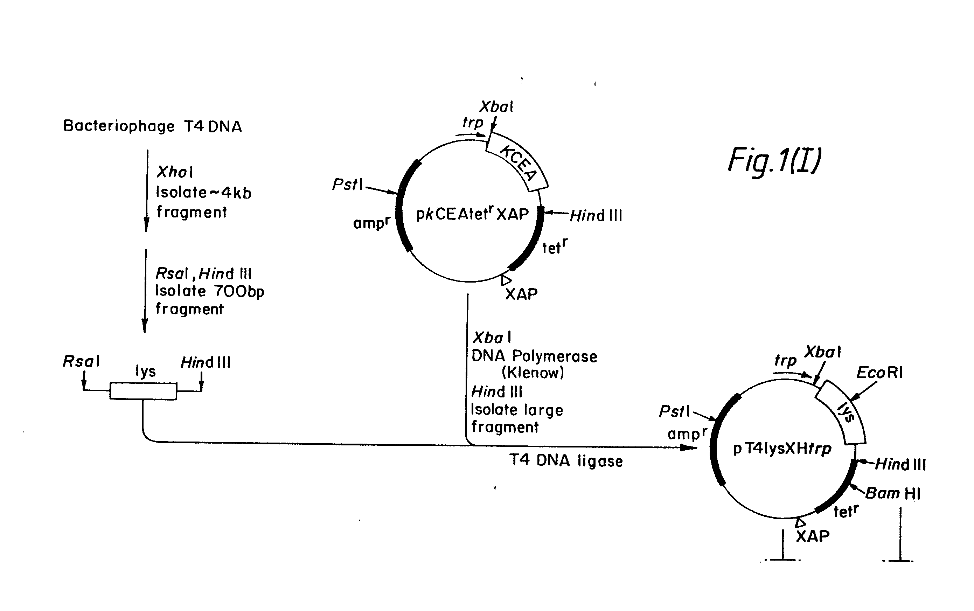

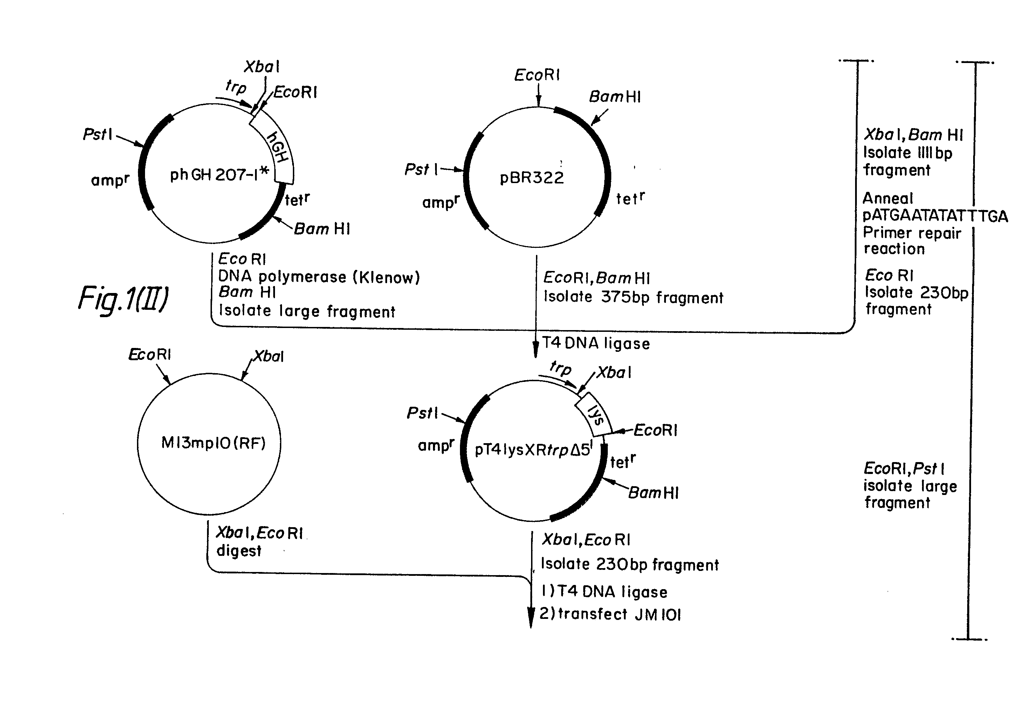

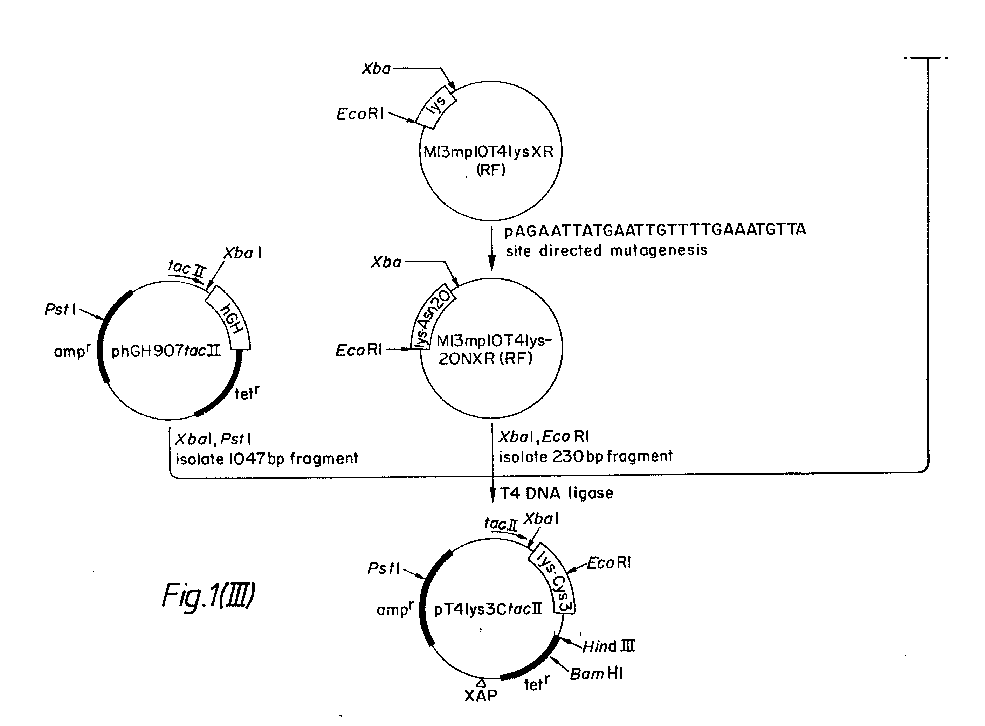

Figure 1 illustrates the construction of the plasmid pT4lys3Ctac II.

Construction of pT4lysXHtrp

A sample of cytosine containing T4 DNA was digested with the restriction endonuclease XhoI and a fragment of about 4,000 base pairs was isolated by agarose gel electrophoresis. The purified fragment was then cleaved with the restriction endonucleases RsaI and HindIII to yield a gel-purified fragment of about 700 base pairs which contained the T4 lysozyme gene. The plasmid pKCEAtetrXAP (6) was cleaved with the restriction endonuclease XbaI, filled in with DNA polymerase I (Klenow fragment) and subsequently digested with the restriction endonuclease HindIII. The gel purified vector fragment was ligated with T4 DNA ligase to the 700 bp fragment to yield the plasmid pT4lysXHtrp.

Construction of pT4lysXRtrp 5

The plasmid phGH207-1*(21) was cleaved with the restriction endonuclease EcoRI and elongated with DNA polymerase I (Klenow fragment), then cleaved with the restriction endonuclease BamHI, and the large vector fragment was purified by gel electrophoresis. The plasmid pT4lysXHtrp was cleaved with the restriction endonucleases XbaI and BamHI to yield a 1111 base pair fragment. A 14-base oligonucleotide primer was used in a primer repair reaction to delete the 97 bases of untranslated T4 DNA on the 5'-end of the lysozyme gene (20). About 0.5 micrograms of the primer was labeled using $^{32}$P-ATP and poly-nucleotide kinase. The gel purified XbaI-BamHI fragment of pT4lysXHtrp was annealed to the labeled primer by heating to 95-100°C for 4 minutes followed by quick freezing. Nucleotide triphosphates were then added to the frozen mixture, which was then allowed to thaw. When the mix was partially thawed, DNA polymerase I (Klenow fragment) was added. The reaction was incubated at 37°C for 1.5 hours. EDTA was added to stop the reaction. The DNA was washed with phenol and chloroform, precipitated and washed with ethanol. The fragment was iden-tified on an acrylamide gel by autoradiography. The identified fragment was eluted from the gel and quantified by scintillation counting. The resulting fragment was then digested with the restriction endonuclease EcoRI to yield a fragment of 230 base pairs which was isolated by gel electrophoresis. The plasmid pBR322 was digested with the restriction endonucleases EcoRI and BamHI and the 375 base pair fragment purified by gel electrophoresis. A three-way ligation reaction was performed with the three fragments to construct the pT4lysXRtrpΔ5' plasmid.

This ligation mixture was used to transform E. coli K-12 294 (ATCC no. 31446). Plasmid DNA was isolated from the resulting transformants and applied to nitrocellulose paper. The paper was treated with $^{32}$P labeled primer. When the nitrocellulose was gradually washed under conditions of increasing hybridization stringency, 37 positive sequences were seen. Six of the most strongly hybridizing positives were picked for M13mp10 sequencing.

8

The plasmid isolated from each of these strongly hybridizing clones was digested with the restriction endonucleases EcoRI and XbaI and the 230 base pair fragment was gel purified. The double standard RF form of the M13mp10 DNA was also digested with EcoRI and XbaI. The 230 base pair fragment was ligated into the digested M13mp10 vector fragment overnight at room temperature. The ligation reaction mixtures were used to transfect E. coli JM101 (ATCC no. 33876), and plaques were picked and sequenced by standard methods. Only one of the positives analyzed by DNA sequencing had the correct sequence. The replicable expression vehicle which contains the EcoRI/XbaI fragment of the correct sequence was designated pT4lysXRtrpΔ5'.

Construction of pT4lys3CtacII

The method of site-directed mutagenesis was used to construct a gene with a cysteine codon replacing an isoleucine codon at the site of the T4 lysozyme gene corresponding to the position 3 amino acid of T4 lysozyme. A 230 base pair fragment of the replicable cloning vehicle pT4lysXRtrpΔ5' containing the 5' half of the lysozyme gene was isolated after treatment of the replicable cloning vehicle with the restriction endonucleases XbaI and EcoRI and was cloned into the phage M13mp10. The synthetic oligomer pAGAATTATGAATTGTTTTGAAATGTTA was used to mutate the isoleucine codon, ATA, to the cysteine codon, TGT. After confirmation by M13 DNA sequencing that the desired mutation had occurred, the mutated DNA fragment was cleaved out of the RF form of M13mp10 with the restriction endonucleases XbaI and EcoRI. This fragment was ligated to the 1047 base pair fragment cleaved from the replicable cloning vehicle phGH907tacII with the restriction endonucleases XbaI and PstI, and to the

9

large fragment of the replicable cloning vehicle pT4LysXHtrp which had been cleaved with EcoRI and PstI, generating the replicable cloning vehicle pT4lys3CtacII. This replicable cloning vehicle was used to transform E. coli D1210 (ATCC no. 31449). The transformants were grown and selected on LB/ampicillin plates. Replicable expression vectors (REVs) were isolated from cells grown from the colonies and analyzed by restriction mapping. Colonies containing REVs with restriction fragments of the expected size, consistent with the desired final construction, were again grown. A SDS polyacrylamide gel with crude cellular protein from each IPTG induced culture indicated the presence or absence of an induced protein of about 19,000 molecular weight. When it was later established that the T4 lysozyme isolated from a culture of these cells contained a cysteine in position 3 the REV isolated from these cells was designated pT4lystacII.

Figure 2 illustrates the mobility of different proteins on a non-reducing SDS polyacrylamide gel.

Figure 3 illustrates the mobility of oxidized, disulfide bonded protein in reverse high performance liquid chromatography (HPLC).

Figure 4 illustrates reverse phase HPLC separation of fragments generated by trypsin digestion of oxidized T4 lysozyme-Cys 3, pre-treated with iodoacetic acid to eliminate disulfide scrambling during digestion.

Figures 5 and 5b illustrate inactivation kinetics of disulfide-crosslinked lysozyme and the corresponding wild type enzyme.

A. Definitions

Hormonal Activity - those biological properties of a molecule which contribute to its action as a hormone including, but not limited to, receptor binding activity, triggering activity, serum lifetime, low immunogenicity and stability toward proteolysis in the circulation.

Receptor Binding Activity - the ability of a molecule to interact in a normal biological mode with its normal biological receptor(s).

Receptor - a molecule or group of associated molecules which function by binding an effector molecule such as a hormone and, upon binding, mediate the transmission of signal to the interior of the organelle which contains on its surface the receptor.

Triggering Activity - the property of an effector molecule such as a hormone which is responsible for transmission of a signal after binding of that effector to its receptor.

Serum Lifetime - the time required for the dissappearance of one half of the amount (as expressed in weight, moles, or activity units) of a molecule from the circulating serum.

Immunogenicity - the ability of a molecule to stimulate an organism or in vitro system to generate antibodies toward that molecule or molecular features of that molecule. As used herein, immunogenicity is unacceptably increased if it leads to failure to gain approval from the Food and Drug Administration.

Site directed mutagenesis - the generation of a specific alteration in a nucleotide sequence, usually the replacement of one base by another at a particular location in the sequence for the purpose of altering the amino acid sequence encoded by the polynucleotide gene. This is usually accomplished using a synthetic oligonucleotide containing the desired change with the sequence to be changed contained on the single-stranded genome of the coliphage M13mp10.

B. Selection of an Appropriate Site to Direct Mutagenesis

The sequence and x-ray crystallograph of the protein chosen for stabilization engineering in accordance with the present invention is first studied. The protein sequence provides information regarding codons encoding for specific amino acids to be mutated. X-ray crystallographs allow, with the aid of computer graphics and an appropriate software package (for example MIDAS), the three-dimensional conformation of the protein to be projected on a computer screen. The crystal structure permits an x-ray crystallograph to be made. This three-dimensional display permits accurate analysis of fundamental physical/chemical properties including the interrelationships of the protein's chemical groups. After careful study, these properties such as bond angles and distances suggest which amino acids may be mutated thus providing the best opportunity for successful protein engineering of disulfide bond formation.

Implementation of this unusual combination of computer graphics and protein chemistry provides a tool for the examination of the dihedral angles which result from protein folding. Once these angles have been thoroughly studied a determination as to the appropriateness of forming a disulfide bond at a specific location is made. The selection of a site in accordance with the present invention provides a newly created cysteine containing mutated protein which will show greater stability than the wild type with retention of substantial activity.

The method of identifying appropriate sites is as follows:

1. The x-ray crystal structure coordinates are searched by a computer program for all pairs of beta carbons in non-adjacent residues which lie within about 5 angstroms of each other but are separated by at least 4 angstroms.

2. Each pair of beta carbons in this list is analyzed by computer graphics for the degree to which it can accommodate a good disulfide bond:

   a. The residues containing the beta carbons are replaced by cysteines in such a way that the atoms common to the wild type residue and inserted cysteine are superimposed.

   b. The side chains of each of the two inserted cysteines are moved, by revolution around the

$C_x$-$C_B$ bonds, until a configuration is reached which allows the sulfur atoms to come within the appropriate bonding distance for sulfur-sulfur bonds, 2.0 angstroms. In some cases an ideal bonding distance is not possible in which case the best possible distance is chosen.

c. The dihedral angles known as $chi_1$, $chi_2$, $chi_3$, $chi'_2$, and $chi'_1$, defined by the positions of the atoms in the side chains, are measured and evaluated with respect to the "allowed" values as determined from x-ray crystal structure data. It will only be possible to get an approximate value of $chi_3$, if the sulfur atoms have not achieved a good bonding length.

3. The pairs from #1 are further assessed by determining how many residues separate the two replacement residues in the linear amino acid sequence. More widely separated pairs are preferred.

4. The pairs are qualitatively ranked in terms of the expected difficulty in making a good disulfide (#2), the number of amino acids by which the replacement residues are separated (#3), the distance of the pair from the active site, when known (the further the better), and the difficulty of introducing cysteines at the positions (in some cases a cysteine already may be present at one of the two sites).

5. The replacements are made in order of the composite rank established in #4.

6. As each mutant protein is constructed, it is assessed for:

   a. Its ability to form the desired disulfide upon oxidation, by methods described herein.

   b. Its ability, when disulfide crosslinked, to function in the same basic way(s) that the wild type functions.

   c. Its ability, if retaining wild type activity, to exhibit enhanced stability, as elaborated in Section E.

7. If no mutants with the desired properties are obtained by No. 1 to 6, repeat the process beginning at step 1 screening all previously unassessed beta carbon pairs separated by about 3.5 to 6 angstroms.

8. If no useful mutants with the desired properties are obtained by No. 1 to 7, repeat the process beginning at step 1 screening all previously unassessed beta carbon pairs separated by about 3.5 to 8 angstroms.

C. Synthesis of a New Gene Coding for Cysteine Containing Mutated Protein by Site Directed Mutagenesis

The single stranded form of an E. coli phage M13mp10, containing the target site for mutation in the gene for the protein to be stabilized, is combined with a synthetic oligonucleotide containing the desired mutation codon that encodes for cysteine. Using standard methods of site directed mutagenesis, this generates an E. coli M13mp10 derivative containing the mutated, cysteine encoding, codon.

The DNA sequence containing the mutated gene is excised from the double stranded replicative form of M13 and used to reassemble an expression plasmid containing the full gene for mutated protein. Mutated protein expression is put under control of the tac II promotor or other equivalent functional promoter which allow high yield expression while optimally permitting fine control of gene expression. The combination of site directed mutagenesis and the genetic engineering techniques which permit the synthesis of a gene for the newly created protein permits the successful introduction of a disulfide bond into protein. Thus, the present invention provides the introduction of a cysteine mutation on the DNA through, for example, site directed mutagenesis on that portion of the DNA molecule which encodes for the wild type residue chosen to be mutated into cysteine.

For the most part, it is necessary to block or remove free sulfhydryl groups which are not intended to take part in disulfide bond formation. This blocking may be effected by use of an oxidizing agent such as sodium tetrathionate which can simultaneously block free sulfhydryl groups and oxidize them or by a suitable sulfhydryl blocking agent such as an alkylating e.g., iodoacetate agent. Offending protein sulfhydryl groups may be removed at the DNA level by site-directed mutagenesis (22). Failure to properly block or remove free sulfhydryl groups could prevent protein stabilization.

D.  Confirmation of Cysteine Substitution and
    Disulfide Bond Formation

Transformants are screened by restriction analysis of isolated plasmids to confirm the structure of the plasmid. Transformants containing plasmids which pass the restriction analysis tests are grown in culture and induced with the inducer isopropyl-beta-D-thiogalactoside. Following induction and resultant genetic expression, the crude cellular protein is tested for the appearance of a new band by electrophoresis on an appropriate SDS polyacrylamide gel. The extracted gene, isolated from one of the clones, is transferred back into M13 to permit sequence confirmation of the mutation at the DNA level.

Mutated protein is purified using standard methods. Titration of the thiol groups of purified protein with Ellman's reagent is run to detect the expected additional cysteine residue(s). Confirmation of the cysteine position may be obtained from amino acid sequencing.

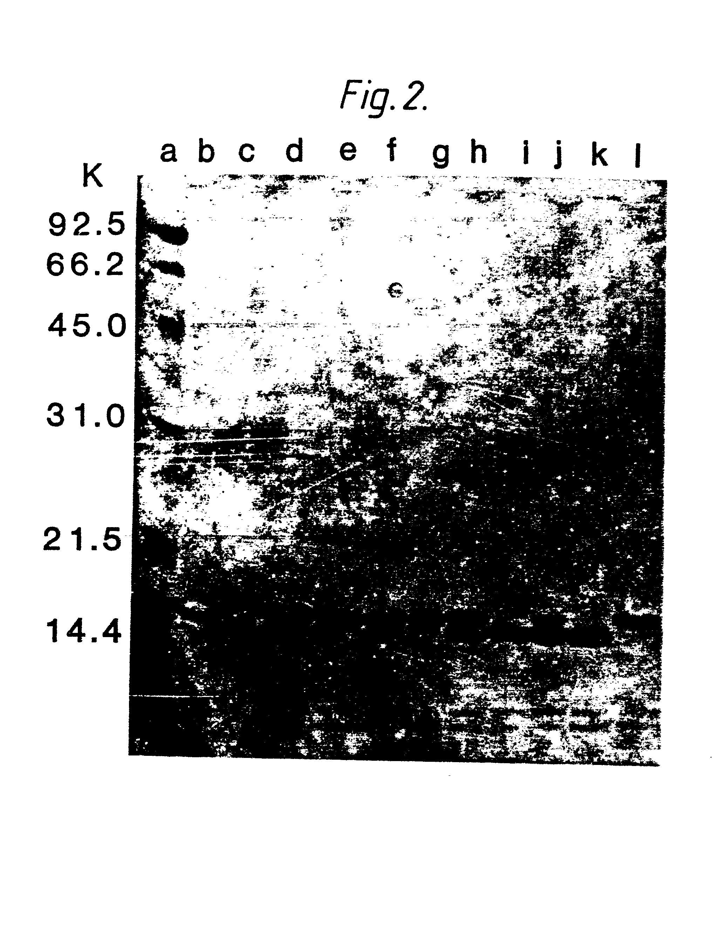

The mobility of the wild type, and oxidized and reduced mutated protein is examined on a non-reducing SDS polyacrylamide gel. Oxidized proteins, containing disulfide bonds often move faster than the reduced sulfhydryl form because of tighter folding induced by the disulfide cross links which decrease the protein's hydrodynamic radius in a denaturing medium. The more highly crosslinked a protein, the faster it moves in a gel. Increasingly favorable reaction conditions (see Figure 2) for disulfide bond formation should produce a protein preparation with increasing proportions of a more mobile protein form. Addition of mild oxidizing agents increases the oxidative strength of the solution, enhancing disulfide bond formation and mobility of the protein in the reaction mixture in subsequent analysis by the electrophoretic gel.

The results of the electrophoretic step may be confirmed by resolving the protein by high pressure liquid chromatographic (HPLC) elution. The HPLC provides greater resolution than an SDS polyacrylamide gel and is especially useful in ascertaining the homogeneity of the induced disulfide bonds. This suggests that all the protein molecules contain disulfide bonds bridging the same two cysteines.

A second column HPLC chromatography step is used to resolve a trypsin digest of the oxidized protein. This step confirms which two cysteines are involved in the newly formed disulfide bond. This is especially important in molecules containing more than two cysteines.

E.    Characterization of Modified Protein Enzyme Activity

The activity of modified enzyme is confirmed by comparing its enzymatic activity on a weight basis with wild type enzymatic activity. The stabilization effect of the induced disulfide bonds can be determined by conducting the enzyme activity assay under denaturing conditions or by assaying under native conditions a protein which has been previously exposed to denaturing conditions. One of the most commercially important denaturing conditions is heat. The thermal stabilizing effect of induced disulfide bonds can be detected by monitoring the effect of exposure of the enzyme to high temperature against its enzyme activity as measured at room temperature.

18

F.    Preferred Embodiment

        To locate sites in T4 lysozyme which might accommodate
a disulfide, its X-ray crystal structure was studied for
coordinates for all beta carbons and for residues separated by
ten or more amino acids lying within six angstroms of each
other.  Working with some of the more closely oriented beta
carbons, an Evans and Sutherland computer graphics system was
used with the software of Langridge and co-workers (10), to
construct disulfide bonds using cysteine residues substituted for
the wild type residues of the protein's primary chain.  In each
case, to obtain the best fit as described in section B above, the
disulfide to be formed was assessed for the extent to which the
sulfur atoms achieved a good bond length, about 2.0 angstroms,
and for the degree to which the five dihedral angles (chi$_1$, chi$_2$,
chi$_3$, chi'$_2$, chi'$_1$) so generated fit within patterns established
by examination of disulfide bonds in proteins of known structure
(3,4).  Each potential disulfide was also assessed for the number
of intervening amino acids in the primary sequence; in theory,
the more sequence a crosslink spans, the greater its contribution
toward conformational stability (3,11).  One of the better
candidates measured by these criteria was the disulfide produced
between cysteines at positions 3 and 97.  Other possible
disulfide crosslink positions were positions 1 and 158, 83 and
112, 1 and 97, and 98 and 152.  Since residue 97 in T4 lysozyme
is already a cysteine, disulfide crosslinking was first attempted
by introducing a cysteine at position 3.

        Using the pT4lysXRtrp 5' plasmid derived as shown in
Figure 1, the synthetic oligonucleotide pAGAATTATGAATTGTTT-
GAAATGTTA and an M13mp10 derivative containing the 5' half of the
T4 lysozyme gene, a derivative of M13mp10 containing the desired

isoluecine (ATA) to cysteine (TGT) mutation was generated by standard methods (2). The lysozyme sequence was excised from the double-stranded replicative form of M13mp10 and used to reassemble a plasmid containing the full, mutated lysozyme gene under the control of the tac II promoter (7,12). Transformants were screened by restriction analysis of isolated plasmid and for the ability of frozen cells to lyse upon thawing. It was observed that E. coli containing the T4 lysozyme in the cytoplasm are stable during fermentation but lyse after a freeze thaw cycle. Several positive clones were checked for the appearance of a new band on an SDS polyacrylamide gel of crude cellular protein, after addition to a growing culture of the tac II inducer isopropyl-beta-D-thiogalactoside. The lysozyme gene from the plasmid, designated pT4Lys3CtacII, isolated from one of the clones was transferred back into M13 and its DNA sequence shown to differ from that of the wild type gene only in the altered third codon.

A colony of E. coli D1210/pT4lys3Ctac II was picked from a culture plate (LB broth/ampicillin) from a fresh transformation and grown overnight at 37° in five ml of LB (19) broth containing 20 micrograms/l ampicillin. This culture was used to inoculate 1 liter of M9 medium (19) containing 20 micrograms/ml ampicillin in a 4 liter shake flask. When the culture reached an $OD_{550}$, of about 2, or late log phase growth, it was induced with the addition of 1mM isopropyl-beta-D-thiogalactoside (19). After one hour the cells were harvested by centrifugation and frozen.

Frozen cells from 0.5 l of culture were lysed by addition to 20 ml 50 mM tris HCl, pH 7.5, 1 mM EDTA, 1 mM 2-mercaptoethanol, 1 mM phenylmethylsulfonyl fluoride on ice.

Two mls of five percent polyethylene imine hydrochloride (pH 7.5) was added with stirring and the suspension centrifuged at 10,000 rpm for 20 minutes in a Sorvall SS-34 rotor. The supernate was passed over a DEAE-cellulose column and washed with 50 mM tris HCl, pH 7.5, 1 mM EDTA, 3 mM 2-mercaptoethanol.

The flowthrough fractions containing 0.3 $OD_{280}$ or higher were pooled and adjusted to pH 5.2 with concentrated acetic acid. This was loaded onto a CM-cellulose column (10 ml bed volume in a 1 cm diameter column at 4° equilibrated with 50 mM NaOAc, pH 5.5, 1 mM EDTA, 3 mM 2-mercaptoethanol. The column was washed with equilibration buffer, then eluted with a linear gradient of 0-0.5 M NaCl in equilibration buffer, total volume 100 ml, at a flow rate of 10 ml/hr. The fractions containing lysozyme activity (19) eluted at a NaCl concentration of about 0.25 M. These fractions contained as the major species a protein shown by SDS polyacrylamide gel electrophoresis to be of molecular weight about 19,000. The yield of protein was 3 milligrams.

Lysozyme purified from cells transformed with pT4Lys3CtacII possessed an additional cysteine residue, as shown by titration of protein thiols with Ellman's reagent (data not shown). In addition, automated Edman degradation of the carboxymethylated protein's N-terminus gave the amino acid sequence MNCFEMLRIDE, consistent with the desired replacement at position 3.

The mobility of an oxidized, disulfide bonded protein on a non-reducing SDS polyacrylamide gel was examined. Each sample was treated with iodocetamide before adding SDS sample loading buffer, to inhibit thiol-disulfide interchange in SDS. Oxidized, disulfide bonded protein migrates faster in the gel than its reduced form, since the disulfides limit SDS-induced unfolding and the resulting increase in a protein's hydrodynamic radius (13,14). Using this method, T4 lysozyme (Ile3->Cys), purified in the presence of beta-mercaptoethanol migrated with the same mobility as the wild type enzyme except for a small amount of a faster moving component not present in the wild type enzyme.

Dialysis to remove beta-mercaptoethanol and raise the pH from 5.5 to 8 generated more of this more compact, presumably disulfide bonded form. On treatment with various agents as elaborated below the relative amount of the more mobile component increased, with increasing oxidative strength. Total conversion to oxidized T4 lysozyme (Ile3->Cys) in this experiment was obtained with sodium tetrathionate (15).

Sodium tetrathionate has the added advantage of forming an intermediate which blocks free sulfhydryl groups. In this instance, sodium tetrathionate blocked the free sulfhydryl of lysozyme which is part of the cysteine residue at position 54. The stabilization described below is attributed not only to the addition of the disulfide, but also, in this case, to the blocking of the sulfhydryl group of residue 54 by reaction with tetrathionate.

Other sulfhydryl blocking groups can be used to provide this necessary function of blocking the sulfhydryl of residue 54. For example, a T4 lysozyme (Ile3->Cys) derivative containing a 3-97 disulfide and a free sulfhydryl at Cys-54 was prepared by oxidizing the derivative in a glutathione redox buffer. Subsequently, this derivative was reacted with 10 mM iodoacetic acid in 8 M. guanidine hydrochloride, 80 mM Tris hydrochloride, pH 8.5, at room temperature for two hours in order to block Cys 54 by alkylation(15). This derivative exhibited the same stability as the sodium tetrathionate oxidized material, as well as the same loss of stability in the presence of reducing agent.

Figure 2 shows the addition of different proteins in the SDS polyacrylamide gel. Each protein was placed in a lane designated a - k as follows: Lane a, low molecular weight standards; lane b, native interferon alpha A; lane c, reduced, carboxymethylated interferon alpha A; lane e, wild type T4 lysozyme; lane g, wild type T4 lysozyme treated with sodium tetrathionate; lane d, T4 lysozyme (Ile3->Cys) in pH 5.5 sodium acetate, 3mM beta-mercaptoethanol; lane f, T4 lysozyme (Ile3-> Cys) 3 dialyzed from pH 5.5 to pH 8, 50 mM Tris.HCl, 1mM EDTA, which solution was used for the following reactions: lane h, T4 lysozme (Ile->8Cys) plus 5 mM GSH (reduced glutathione) and 5 mM GSSG (oxidized glutathione) (16 hrs, RT); lane i, T4 lysozyme (Ile3->Cys) plus 2.5 mM Cu(OAc)$_2$ 16 hrs, RT; lane j, T4 lysozyme (Ile3->Cys) plus 5 mM GSSG (16 hrs, RT); lane k, T4 lysozyme (Ile3->Cys), 10 mM sodium tetrathionate (16 hrs, RT).

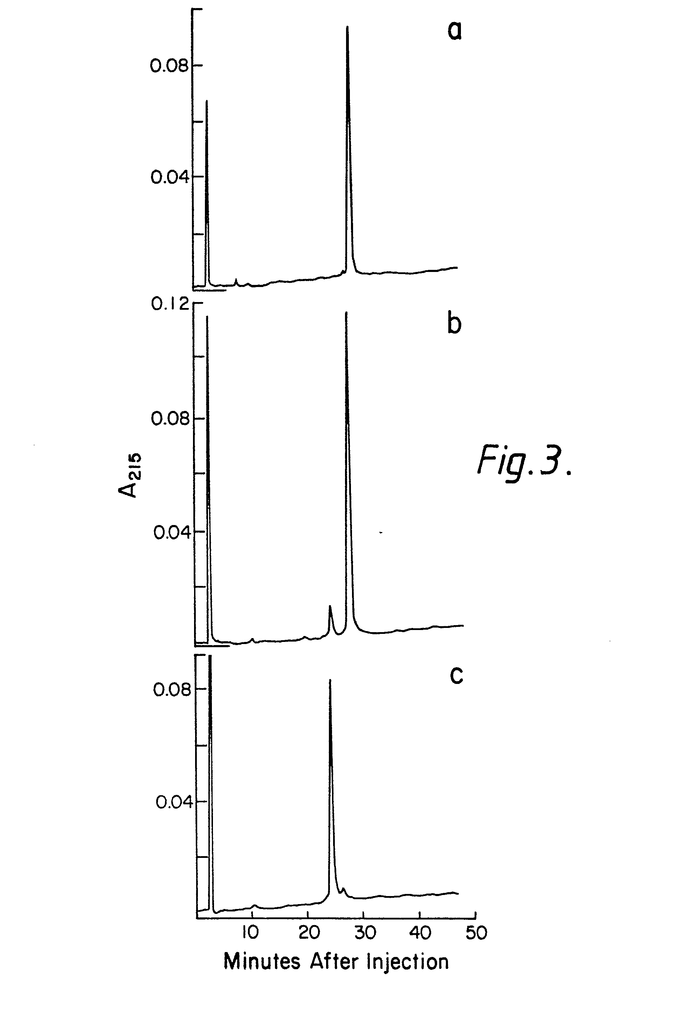

The mobility of the oxidation product in reverse phase high performance liquid chromatography also indicated the presence of a disulfide bond (Fig. 3). Samples were loaded on a Vydak 218-TP546 C$_{18}$, column (Rainin) and eluted at 1 ml/min with a linear gradient of 37 to 52 percent acetonitrile in 0.1 percent aqueous trifluoroacetic acid over 45 minutes. Increased crosslinking favors earlier elution in this chromatography, as observed in experiments with alpha interferon (16), presumably by reducing the interaction of buried hydrophobic residues with the resin under the denaturing conditions of the chromatography. In addition, Fig. 3 suggests that the oxidation product is a single molecular form; that is, there is no evidence for the formation of molecules with other disulfide linkages.

The added cysteine at position 3 brought to three the total cysteines in the protein. To determine which of the three possible pairings were obtained in the oxidation, analysis using trypsin fragments of the oxidized protein was undertaken. The oxidation was repeated on 500 micrograms of the protein and the product purified from the reaction buffer and minor protein contaminants by high performance liquid chromatography on a TSK-3000 molecular sizing column. The disulfide bond in this product was localized by HPLC/tryptic mapping (17).

The sample was alkylated in urea with iodoacetic acid then dialyzed into 100 mM Tris HCl, pH 8, 1mM EDTA, 0.1 M NaCl. The sample was heated to 95°C for 5 minutes, then cooled to 37°C and trypsin (1:40 enzyme/substrate ratio) added; after 3 hours an equivalent amount of trypsin was added; after 3 more hours reaction was stopped by adjusting to pH 2. Samples were chromatographed in the system described in Figure 2 except the gradient was from 4-64 percent acetonitrile in 90 min.

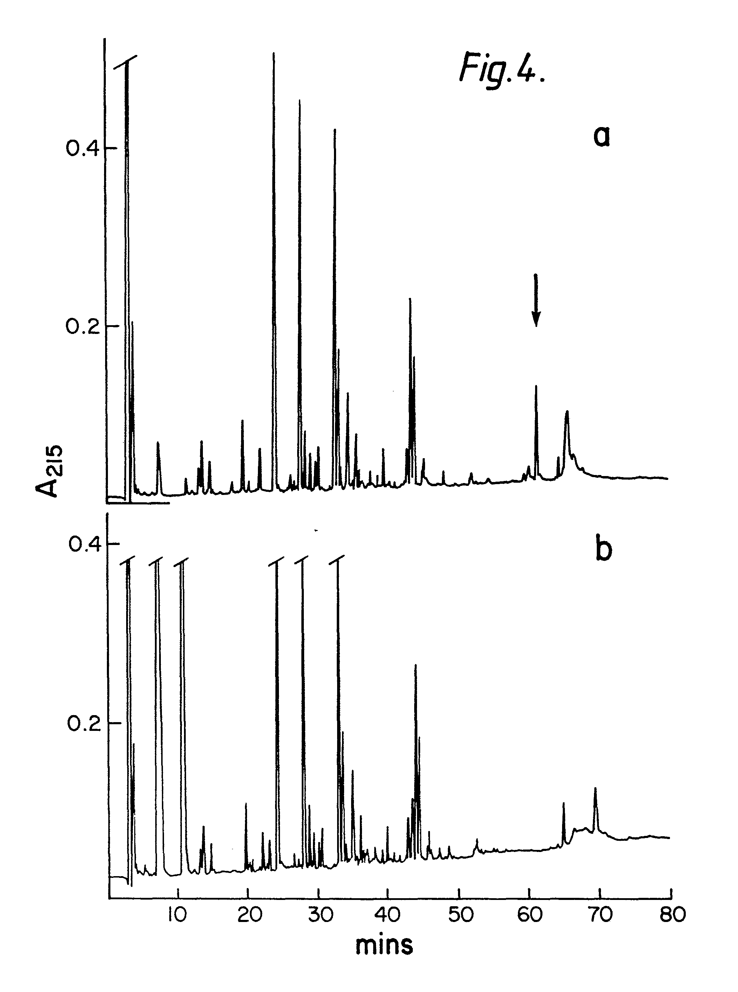

Figure 4 shows the reverse phase HPLC separation of fragments generated by trypsin digestion of oxidized T4 lysozyme (Ile3->Cys) which had been previously treated with iodoacetic acid to eliminate disulfide scrambling during the digestion. By comparing the chromatogram of the untreated digest with that of an aliquot treated with reducing agent, identification of a candidate for the peptide containing the disulfide bond was possible. Amino acid compositional analysis (not shown) was in good agreement with one of the three possible disulfide peptides, and the amino acid sequence of this peptide contained, as expected, two tracks. These were consistent with the sequences MN-FEML- and -ALINMVFQMG (where "-" indicates an undetected amino

24

acid) arising from a peptide generated from a disulfide bond between Cys3 and Cys97 of T4 lysozyme (Ile3->Cys).

In the turbidity assay at 22 degrees (18) both reduced and oxidized, disulfide-containing T4 lysozyme (Ile3->Cys) exhibited the same specific activity as wild-type T4 lysozyme.

The specific activity of lysozyme was studied using the kinetics of inactivation of lysozyme derivatives. Lysozymes were dissolved at three micrograms/milliliter in 100mM potassium phosphate, pH 6.5, 0.1M NaCl, 1 mM EDTA and 100 microliter aliquots added to pyrex tubes and equilibrated at 67°C. At various times samples were plunged into ice, then later diluted and assayed in the turbidity assay at 22°C.

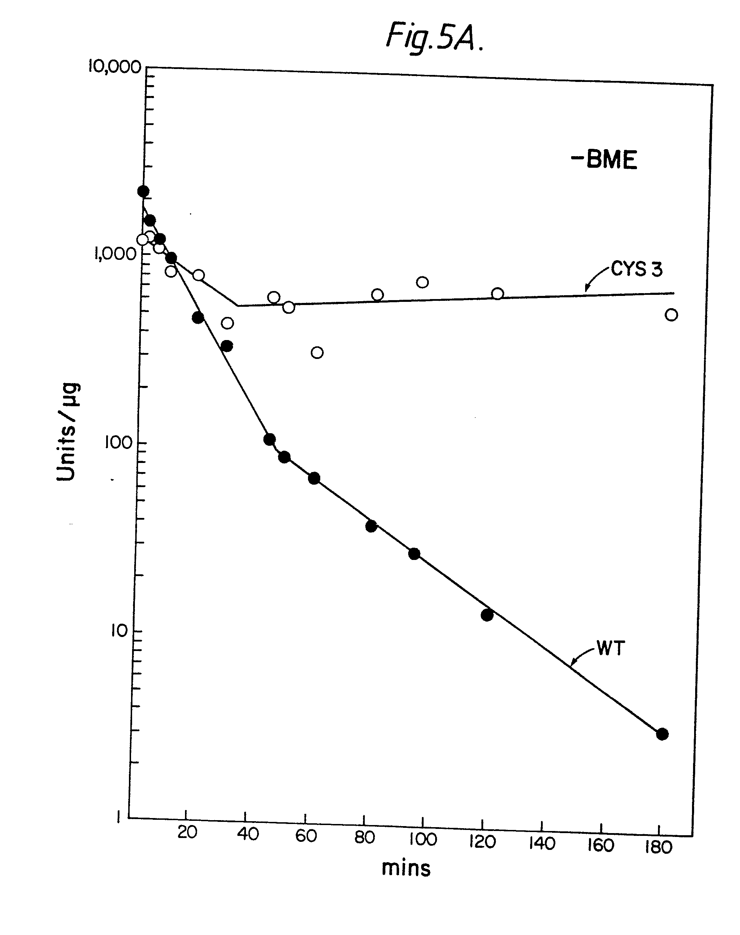

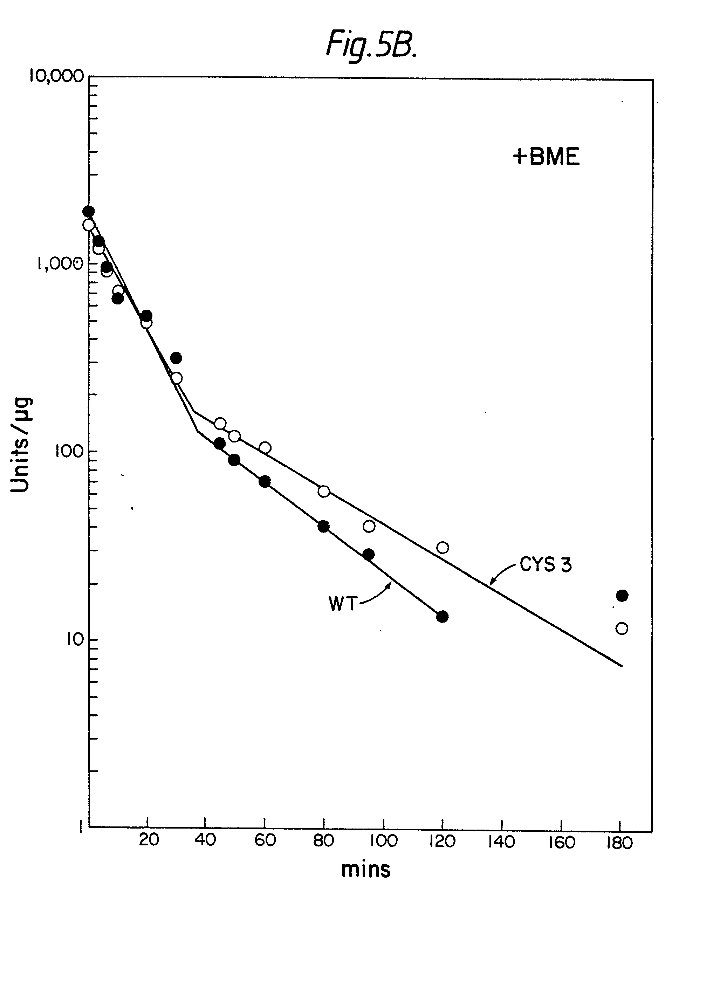

Figure 5a shows that at 67°C the initial rate of inactivation of the disulfide crosslinked lysozyme is less than half of the wild type enzyme. It also shows that, while the wild type enzyme activity continues to decay over three log units, T4 lysozyme (Ile3->Cys) decays to a plateau approximately 50 percent the starting activity, where it remains regardless of the length of incubation at 67°C up to 180 mins. Also shown in Figure 5b are the inactivation kinetics for both wild type and disulfide crosslinked lysozyme in reducing agent, which suggests that it is specifically the disulfide bond, and not merely the cysteine replacement at position 3, which is responsible for the stablization effect.

It is understood that various other modifications will be apparent to and can readily be made by those skilled in the art without departing from the scope and spirit of this invention. Accordingly, it is not intended that the scope of the claims appended hereto be limited to the description as set forth herein, but rather that the claims be construed as encompassing

25

all the features of patentable novelty which reside in the present invention, including all features which would be treated as equivalents thereof, by those skilled in the art to which this invention pertains.

0155832

26

BIBLIOGRAPHY

The following references are cited in the foregoing specification by parenthetical numerals corresponding to those listed below. All of these references are hereby incorporated by reference.

1. Wetzel, R., et al., The Peptides-Analysis, Synthesis, Biology, 5, 1-64.

2. Zoller, M. J., et al., Methods in Enzymology, 100, 468-500.

3. Thornton, J. M., J. Mol. Biol., 151, 261-287.

4. Richardson, J., Advs. Prot. Chem., 34, 167-339.

5. Villafranca, J. E., et al., Science, 222, 782-788.

6. Cabilly, Shmuel, et al., Generation of Antibody Activity from Immunoglobulin Polypeptide Chains Produced in Escherichia coli Proc. Natl. Acad. Sci. (USA), 81, 3273.

7. De Boer, H., et al., From Gene to Protein: Translation into Biotechnology; Miami Winter Symposia, 19, 309-327.

8. Goeddel, D. V., et al., Proc. Natl. Acad. Sci. (USA), 76, 106-110.

9. Messing, J., Methods in Enzymology, 101, 20-78.

10. Langridce, R., et al., Science, 211, 661-11. Anfinsen, C. E., et al., Advs. Protein Chem., 29 205-300.

12. De Boer, H. A., Proc. Natl. Acad. Sci. (USA), 80 21-25.

13. Scheele, G., et al., J. Biol. Chem., 257, 12277-12282.

14. Pollitt, S., et al., J. Bact., 153, 27-32.

15. Means, G. E. et al., Chemical Modification of Proteins, (1971), Holden-Day.

16. Wetzel, R., et al., The Biology of the Interferon System 1983, Elsevier, 101-112.

17. Wetzel, R., Nature, 289, 606-607.

18. Tsugita, A., et al., J. Biol. Chem., 243, 391-397.

19. Miller, J. H., Experiments in Molecular Genetics, (1972), Cold Spring Harbor Laboratory.

20. Goeddel, D. V., et al., Nucleic Acids Res., 8, 4057-4074.

21. Gray, et al., Bio/Technology, 2, 161-165.

22. Wang, A., et al., Science, 224, 1431-1433.

## CLAIMS

1. An active protein which contains at least one disulfide bond formed between cysteine residues in addition to those which may exist in the naturally occurring form of said protein.

2. The protein of claim 1 wherein said protein has no naturally occurring disulfide bonds.

3. The protein of claim 1 wherein said protein has naturally occurring disulfide bonds.

4. The protein of any one of the preceding claims wherein the beta carbons of the cysteines forming the additional disulfide bond are separated by a distance of 3.5 to 8 angstroms.

5. The protein of any one of claims 1, 2 and 3 wherein at least one cysteine residue is introduced to replace a wild type residue, whose alpha carbons are separated by a distance of 3.5 to 10 angstroms.

6. The protein of any one of the preceding claims wherein said protein retains activity against inactivating forces.

7. The protein of claim 6 wherein said inactivating forces are selected from heat, pH extremes, presence of denaturing chemicals, oxidation, hydrolysis, temperature variations or degradative enzymes.

8. The protein of claim 1 wherein said active protein is a hormone or an enzyme.

9. The protein of claim 8 wherein said protein facilitates lysis of microorganisms.

10. The protein of claim 9 wherein said protein is a lysozyme.

11. The protein of claim 10 wherein said protein is T4 lysozyme.

12. The protein of claim 11 wherein said T4 lysozyme contains cysteine substitution for isoleucine at position three.

13. DNA encoding the protein of claim 1.

14. A plasmid containing the DNA of claim 13.

15. A plasmid having DNA encoding in a protein cysteines which can be oxidized to form at least one non-naturally occurring disulfide bond.

16. A process for producing an active protein which contains at least one disulfide bond in addition to those which may exist in the naturally occurring form of said protein comprising the introduction of at least one disulfide bond between a naturally occurring cysteine and a cysteine which has been introduced in place of a wild type residue.

17. A process for producing an active protein which contains at least one disulfide bond in addition to those which may exist in the naturally occurring form of said protein comprising the introduction of at least one disulfide bond between two cysteine residues each of which has been introduced in place of a wild type residue.

18. A process for producing an active protein which contains at least one disulfide bond in addition to those which may exist in the naturally occurring form of said protein comprising the introduction of at least one disulfide bond between two wild type residues both of which are cysteine.

19. The plasmid pT4Lys3CtacII.

20. A culture comprising cells harboring a plasmid of claim 14 or claim 15 capable of producing a protein of any one of claims 1 to 12.

32

CLAIMS

1.   A process for producing an active protein which contains at least one disulfide bond in addition to those which may exist in the naturally occurring form of said protein comprising the introduction of at least one disulfide bond between a naturally occurring cysteine and a cysteine which has been introduced in place of a wild type residue.

2.   A process for producing an active protein which contains at least one disulfide bond in addition to those which may exist in the naturally occurring form of said protein comprising the introduction of at least one disulfide bond between two cysteine residues each of which has been introduced in place of a wild type residue.

3.   A process for producing an active protein which contains at least one disulfide bond in addition to those which may exist in the naturally occurring form of said protein comprising the introduction of at least one disulfide bond between two wild type residues both of which are cysteine.

4.   The process of any one of claims 1, 2 and 3 wherein said protein has no naturally occurring disulfide bonds.

5. The process of any one of claims 1, 2 and 3 wherein said protein has naturally occurring disulfide bonds.

6. The process of any one of the preceding claims wherein the beta carbons of the cysteines forming the additional disulfide bond are separated by a distance of 3.5 to 8 angstroms.

7. The process of any one of claims 1 to 5 wherein at least one cysteine residue is introduced to replace a wild type residue, whose alpha carbons are separated by a distance of 3.5 to 10 angstroms.

8. The process of any one of the preceding claims wherein said protein retains activity against inactivating forces.

9. The process of claim 8 wherein said inactivating forces are selected from heat, pH extremes, presence of denaturing chemicals, oxidation, hydrolysis, temperature variations or degradative enzymes.

10. The process of any one of claims 1, 2 and 3 wherein said active protein is a hormone or an enzyme.

11. The process of claim 10 wherein said protein facilitates lysis of microorganisms.

12. The protein of claim 11 wherein said protein is a
lysozyme.

13. The protein of claim 12 wherein said protein is T4
lysozyme.

14. The process of claim 13 wherein said T4 lysozyme
contains cysteine substitution for isoleucine at position
three.

## Fig.1(I)

Bacteriophage T4 DNA

↓ XhoI
Isolate ~4kb fragment

↓ RsaI, HindIII
Isolate 700bp fragment

RsaI — lys — HindIII

pkCEAtet^r XAP
trp — XbaI — KCEA — HindIII — tet^r — XAP — ampr — PstI

↓ XbaI
DNA Polymerase (Klenow)
HindIII
Isolate large fragment

↓ T4 DNA ligase

pT4lysXHtrp
trp — XbaI — lys — EcoRI — HindIII — Bam HI — tet^r — XAP — ampr — PstI

Fig.1(II)

0155832

Fig.1(III)

Fig. 2.

Fig.3.

Fig.4.

a

b

Fig.5A.

# Fig.5B.

+BME

CYS 3

WT

Units/µg

mins